# EUROPEAN PATENT SPECIFICATION

(11) **EP 4 197 472 B1**
(45) Date of publication and mention of the grant of the patent: **30.09.2026**
(21) Application number: 22214044.4
(22) Date of filing: 16.12.2022
(51) Int. Cl.: A61B 18/14

(54) **CATHETER END EFFECTOR WITH LATERALLY PROJECTING BODY**
KATHETERENDEFFEKTOR MIT SEITLICH VORSTEHENDEM KÖRPER
EFFECTEUR TERMINAL DE CATHÉTER AVEC CORPS À SAILLIE LATÉRALE

(30) Priority: 17.12.2021 US 202163290771 P; 30.11.2022 US 202218071769
(43) Date of publication of application: 21.06.2023
(73) Proprietor: Biosense Webster (Israel) Ltd., Yokneam, 2066717 (IL)
(72) Inventor: Ebrahimi, Babak, Irvine 92618 (US); Van Niekerk, Pieter E., Irvine 92618 (US); Knudson, John C., Irvine 92618 (US)
(74) Representative: Carpmaels & Ransford LLP

(56) References cited:
- WO-A1-2017/070559
- WO-A1-2021/011289
- US-A1- 2021 059 745
- US-A1- 2021 121 231

## Description

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by application of energy (e.g., radiofrequency (RF) energy), it may be possible to cease or modify the propagation of unwanted electrical signals from one portion of the heart to another. The ablation process may provide a barrier to unwanted electrical pathways by creating electrically insulative lesions or scar tissue that effectively block communication of aberrant electrical signals across the tissue.

In some procedures, a catheter with one or more RF electrodes may be used to provide ablation within the cardiovascular system. The catheter may be inserted into a major vein or artery (e.g., the femoral artery) and then advanced to position the electrodes within the heart or in a cardiovascular structure adjacent to the heart (e.g., the pulmonary vein). The one or more electrodes may be placed in contact with cardiac tissue or other vascular tissue and then activated with RF energy to thereby ablate the contacted tissue. In some cases, the electrodes may be bipolar. In some other cases, a monopolar electrode may be used in conjunction with a ground pad or other reference electrode that is in contact with the patient that is in contact with the patient. Irrigation may be used to draw heat from ablating components of an ablation catheter; and to prevent the formation of blood clots near the ablation site.

Examples of ablation catheters are described in U.S. Pat. No. 10,743,932, entitled "Integrated Ablation System using Catheter with Multiple Irrigation Lumens," issued August 18, 2020, U.S. Pat. No. 10,660,700, entitled "Irrigated Balloon Catheter with Flexible Circuit Electrode Assembly," issued May 26, 2020, U.S. Pub. No. 2018/0071017, entitled "Ablation Catheter with a Flexible Printed Circuit Board," published March 15, 2018, U.S. Pat. No. 10,702,177, entitled "Catheter with Bipole Electrode Spacer and Related Methods," issued July 7, 2020, U.S. Pat. No. 10,130,422, entitled "Catheter with Soft Distal Tip for Mapping and Ablating Tubular Region," issued November 20, 2018, U.S. Pat. No. 8,956,353, entitled "Electrode Irrigation Using Micro-Jets," issued February 17, 2015, and U.S. Pat. No. 9,801,585, entitled "Electrocardiogram Noise Reduction," issued October 31, 2017.

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998. Examples of EP mapping catheters are described in U.S. Pat. No. 9,907,480, entitled "Catheter Spine Assembly with Closely-Spaced Bipole Microelectrodes," issued March 6, 2018, U.S. Pat. No. 10,130,422, entitled "Catheter with Soft Distal Tip for Mapping and Ablating Tubular Region," issued November 20, 2018, and U.S. Pat. No. 10,702,177, entitled "Catheter with Bipole Electrode Spacer and Related Methods," issued July 7, 2020.

In addition to using EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued November 1, 2016, and various other references that are cited herein.
In WO 2021/011289 A1, there is described an apparatus including a catheter shaft assembly and an end effector. The catheter shaft assembly includes an outer sheath with a distal end. The end effector is associated with a distal end of the catheter shaft assembly. The end effector includes a plurality of leaflets.
In US 2021/121231 A1, there is described an apparatus including a catheter shaft assembly and an end effector. The end effector includes a plurality of strips and a plurality of electrodes. The strips are configured to fit within an outer sheath of the catheter shaft assembly in a first configuration. The strips are configured to expand outwardly away from a longitudinal axis defined by the catheter shaft assembly in a second configuration when exposed distally relative to the distal end of the outer sheath. The electrodes are positioned on at least some of the strips. The electrodes are positioned relative to each other such that groups of four of the electrodes define a substantially square configuration.
In US 2021/059745 A1, there is described an apparatus including a catheter assembly and an end effector. The catheter assembly includes an outer sheath with a distal end. The end effector is associated with a distal end of the catheter assembly. The end effector includes a panel assembly with microelectrodes for electrophysiological (EP) mapping. The microelectrodes are configured in a matrix within the panel assembly and provide multiple points of contact with the target tissue for EP mapping.
In WO 2017/070559 A1, there is described an integrated electrode structure comprising a catheter shaft comprising a proximal end and a distal end, the catheter shaft defining a catheter shaft longitudinal axis. A flexible tip portion can be located adjacent to the distal end of the catheter shaft, the flexible tip portion comprising a flexible framework. A plurality of microelectrodes can be disposed on the flexible framework and can form a flexible array of microelectrodes adapted to conform to tissue. A plurality of conductive traces can be disposed on the flexible framework, each of the plurality of conductive traces can be electrically coupled with a respective one of the plurality of microelectrodes.

US 2014/200639 A1 discloses further background prior art.

While several catheter systems have been made and used, it is believed that no one prior to the inventors has made or used the invention described in the appended claims.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient;
FIG. 2A depicts a perspective view of the catheter assembly of FIG. 1, with an end effector of the catheter in a proximal position relative to an outer sheath of the catheter;
FIG. 2B depicts a perspective view of the catheter assembly of FIG. 1, with the end effector in a distal position relative to the outer sheath;
FIG. 3 depicts a perspective view of the end effector of FIG. 2B, with layers of the end effector separated from each other;
FIG. 4 depicts a top plan view of an alternative end effector that may be incorporated into the catheter assembly of FIG. 1;
FIG. 5A depicts a top plan view of another alternative end effector that may be incorporated into the catheter assembly of FIG. 1, with leaves of the end effector in a first configuration;
FIG. 5B depicts a top plan view of the end effector of FIG. 5A, with leaves of the end effector in a second configuration; and
FIG. 6 depicts a top plan view of another alternative end effector that may be incorporated into the catheter assembly of FIG. 1.

### DETAILED DESCRIPTION

The invention provides an apparatus according to claim 1. Further embodiments of the invention are described in the dependent claims. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

As used herein, the terms "generally," "substantially," "about," or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 99%. In addition, as used herein, the terms "patient," "host," "user," and "subject" refer to any human or animal subject and are not intended to limit the systems or methods to human use, although use of the subject invention in a human patient represents a preferred embodiment.

### I. Overview of Example of a Catheter System

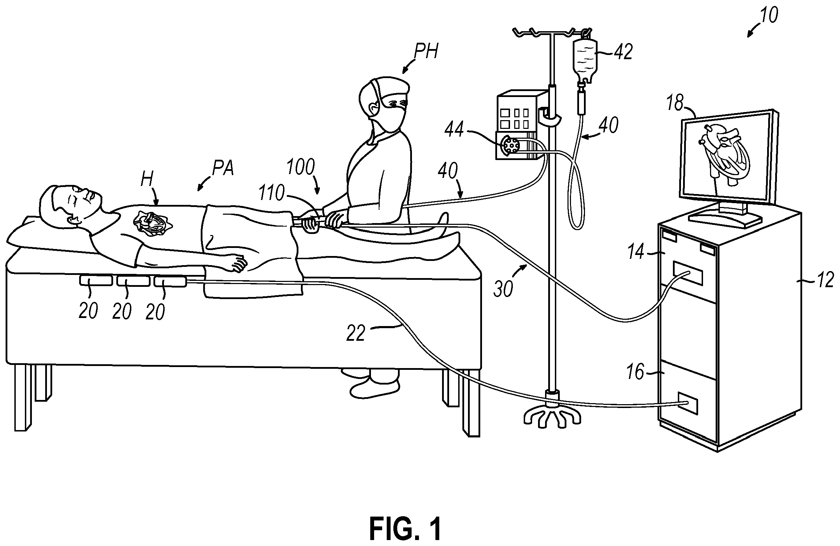

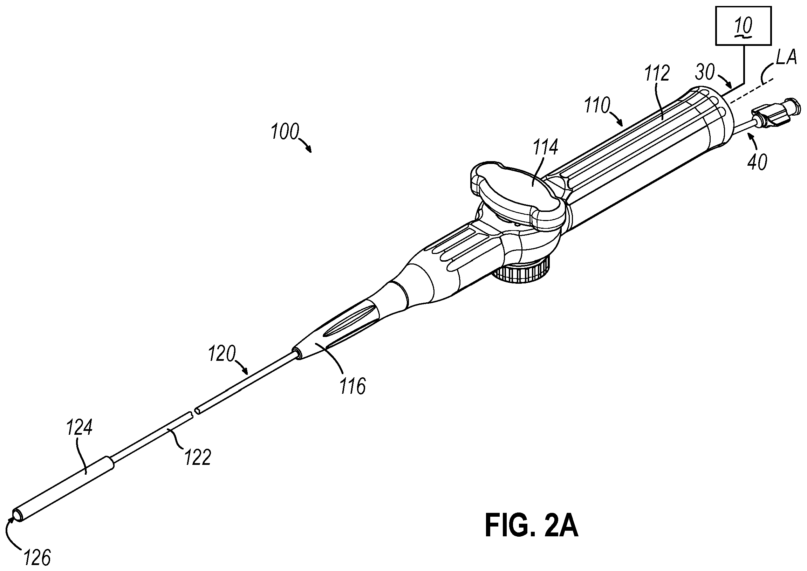

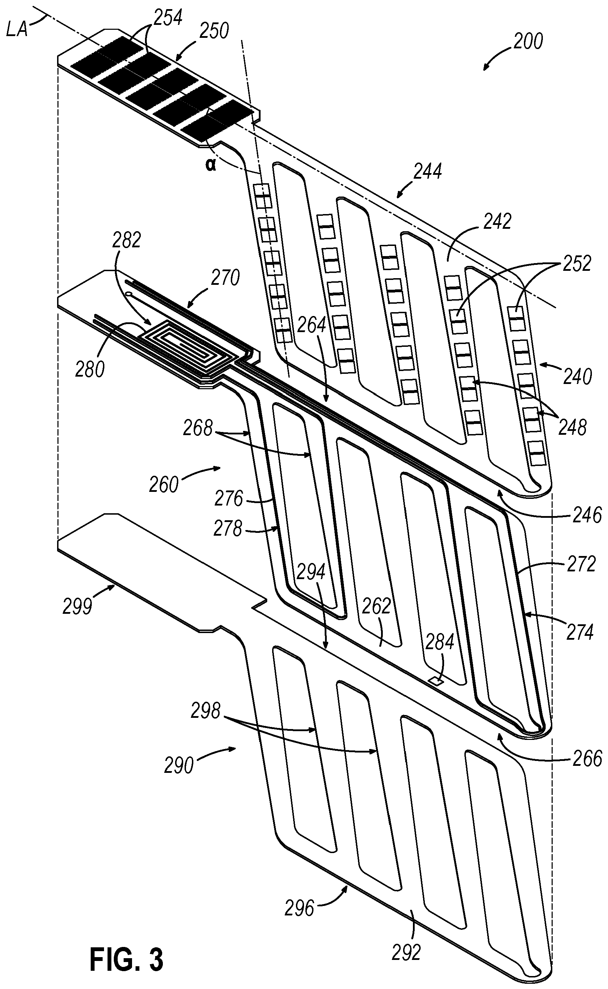

FIG. 1 shows an exemplary medical procedure and associated components of a cardiac catheter system that may be used to provide EP mapping or cardiac ablation as referred to above. In particular, FIG. 1 shows a physician (PH) grasping a handle assembly (110) of a catheter assembly (100), with an end effector (200) (shown in FIG. 2A and FIG. 3 but not shown in FIG. 1) of a flexible catheter (120) (shown in FIGS. 2A-2B but not shown in FIG. 1) of catheter assembly (100) disposed in a patient (PA) to map potentials in tissue or ablate tissue in or near the heart (H) of the patient (PA). As shown in FIGS. 2A-2B, catheter assembly (100) includes handle assembly (110), catheter (120) extending distally from handle assembly (110), end effector (200) located at a distal end of catheter (120), and a deflection drive actuator (114) associated with handle assembly (110). Deflection drive actuator (114) is rotatable relative to a casing (112) of handle assembly (110) to thereby deflect end effector (140) and a distal portion of catheter (120) away from a central longitudinal axis (LA) defined by a proximal portion of catheter (120). Various suitable components that may be coupled with deflection drive actuator (114) and catheter (120) to provide such functionality will be apparent to those skilled in the art in view of the teachings herein.

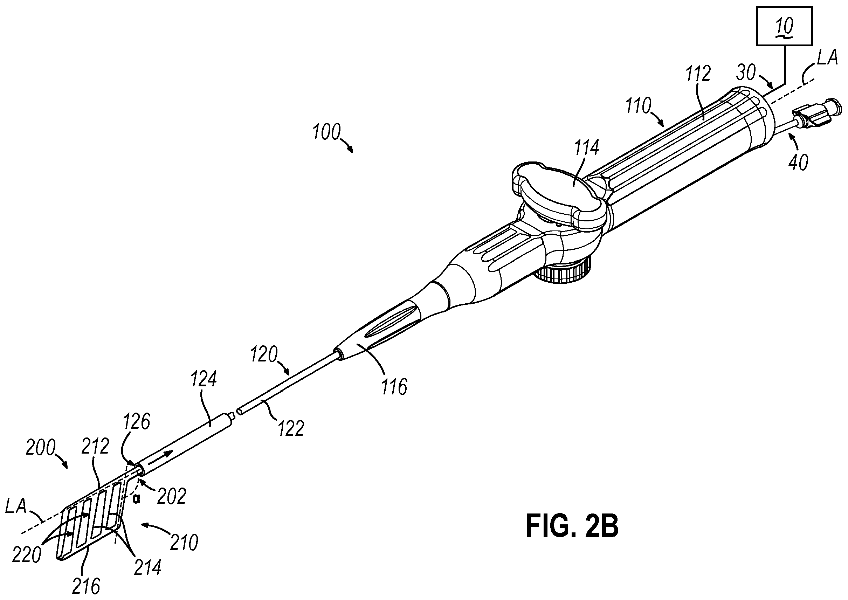

Catheter (120) includes an elongate flexible shaft (122) and an outer sheath (124). Shaft (122) is coaxially and slidably disposed within outer sheath (124). End effector (200) is positioned at the distal end of shaft (122). The proximal end of catheter (120) extends distally from a nozzle member (116) of handle assembly (110). In some versions, outer sheath (124) is an integral component of catheter (120). In some other versions, sheath (124) is a component of another instrument; and catheter (120) is inserted into sheath (124). In either scenario, shaft (122) and an outer sheath (124) may transition between two arrangements, including a first arrangement as shown in FIG. 2A where outer sheath (124) is distally positioned in relation to shaft (122), such that end effector (200) is contained within outer sheath (124); and a second arrangement as shown in FIG. 2B where outer sheath (124) is proximally positioned in relation to shaft (122), such that end effector (200) is exposed relative to outer sheath (124). In some versions, shaft (122) translates relative to handle assembly (110) to achieve the different longitudinal positioning relative to outer sheath (124). In some other versions, outer sheath (124) translates relative to handle assembly (110) to achieve the different longitudinal positioning relative to shaft (122).

Catheter assembly (100) is coupled with a guidance and drive system (10) via a cable (30). Catheter assembly (100) is also coupled with a fluid source (42) via a fluid conduit (40), though this is optional. A set of field generators (20) are positioned underneath the patient (PA) and are also coupled with guidance and drive system (10) via a cable (22). Guidance and drive system (10) of the present example includes a console (12) and a display (18). Console (12) includes a first driver module (14) and a second driver module (16). First driver module (14) is coupled with catheter assembly (100) via cable (30). In some variations, first driver module (14) is operable to receive EP mapping signals obtained via pairs of electrodes (252) of end effector (200) as described in greater detail below. Console (12) includes a processor (not shown) that processes such EP mapping signals and thereby provides EP mapping as is known in the art. In addition, or in the alternative, first driver module (14) may be operable to provide RF power to ablation electrodes (not shown) of end effector (200) to thereby ablate tissue.

In some versions, first driver module (14) is also operable to receive position indicative signals from one or more position sensor coils (274, 278, 280) in end effector (200), as will be described in greater detail below. In such versions, the processor of console (12) is also operable to process the position indicative signals from position sensor coils (274, 278, 280) to thereby determine the position of end effector (200) of catheter (120) within the patient (PA). Other components and techniques that may be used to generate real-time position data associated with end effector (200) may include wireless triangulation, acoustic tracking, optical tracking, inertial tracking, and the like.

Second driver module (16) is coupled with field generators (20) via cable (22). Second driver module (16) is operable to activate field generators (20) to generate an alternating magnetic field around the heart (H) of the patient (PA). For instance, field generators (20) may include coils that generate alternating magnetic fields in a predetermined working volume that contains the heart (H).

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from position sensor coils (274, 278, 280) of end effector (200). For instance, as end effector (200) of catheter (120) moves within the patient (PA), the corresponding position data from position sensor coils (274, 278, 280) may cause the processor of console (12) to update the patient anatomy views in display (18) in real time to depict the regions of patient anatomy around end effector (200) as end effector (200) moves within the patient (PA). Moreover, the processor of console (12) may drive display (18) to show locations of aberrant conductive tissue sites, as detected via EP mapping with end effector (200). By way of example only, the processor of console (12) may drive display (18) to superimpose the locations of aberrant conductive tissue sites on the images of the patient's anatomy, such as by superimposing an illuminated dot, a crosshair, or some other form of visual indication of aberrant conductive tissue sites.

The processor of console (12) may also drive display (18) to superimpose the current location of end effector (200) on the images of the patient's anatomy, such as by superimposing an illuminated dot, a crosshair, a graphical representation of end effector (200), or some other form of visual indication. Such a superimposed visual indication may also move within the images of the patient anatomy on display (18) in real time as the physician moves end effector (200) within the patient (PA), thereby providing real-time visual feedback to the operator about the position of end effector (200) within the patient (PA) as end effector (200) moves within the patient (PA). The images provided through display (18) may thus effectively provide a video tracking the position of end effector (200) within a patient (PA), without necessarily having any optical instrumentation (i.e., cameras) viewing end effector (200). In the same view, display (18) may simultaneously visually indicate the locations of aberrant conductive tissue sites detected through the EP mapping. The physician (PH) may thus view display (18) to observe the real time positioning of end effector (200) in relation to the mapped aberrant conductive tissue sites and in relation to images of the adjacent anatomical structures in the patient (PA).

Fluid source (42) of the present example includes a bag containing saline or some other suitable irrigation fluid. Conduit (40) includes a flexible tube that is further coupled with a pump (44), which is operable to selectively drive fluid from fluid source (42) to catheter assembly (100). In some variations, conduit (40), fluid source (42), and pump (44) are omitted entirely. In versions where these components are included, end effector (200) may be configured to communicate irrigation fluid from fluid source (42) to the target site in the patient. Such irrigation may be provided in accordance with the teachings of any of the various patent references cited herein; or in any other suitable fashion as will be apparent to those skilled in the art in view of the teachings herein.

### II. Example of End Effector with Multi-Layered Flex Circuit Sub-Assemblies

Some kinds of EP mapping end effectors may be particularly configured for use in the chambers of a heart (H), where the end effector may be pressed against the walls defining the chambers of the heart (H) in order to pick up electrocardiogram signals from the tissue. Some other kinds of EP mapping end effectors may be particularly configured for use in the pulmonary vein, where the end effector will be pressed against the wall of the pulmonary vein in order to pick up electrocardiogram signals from the tissue. Those skilled in the art will recognize that the geometry of the wall of the pulmonary vein is substantially different from the walls defining the chambers of the heart (H). For instance, the wall of the pulmonary vein has a generally circular cross-section, with a relatively small radius of curvature. By contrast, the walls defining the chambers of the heart (H) may have generally flat regions and curved regions with a radius of curvature that is larger than the radius of curvature associated with the pulmonary vein.

Thus, due to the differences in geometries of the pulmonary vein and the walls defining the chambers of the heart (H), an EP mapping end effector that is specifically configured for use in a pulmonary vein may not be suitable for use in the chambers of the heart (H). Similarly, an EP mapping end effector that is specifically configured for use in the chambers of the heart (H) may not be suitable for use in the pulmonary vein. It may therefore be desirable to provide an EP mapping end effector that is suitable for use in both the pulmonary vein and in the chambers of the heart (H). An example of such an end effector (200) is described in greater detail below.

As shown in FIG. 2A, end effector (200) is deformable to fit entirely within outer sheath (124) when outer sheath (124) is distally positioned in relation to shaft (122). As shown in FIG. 2B, end effector (200) is also configured to achieve an expanded configuration when outer sheath (124) is proximally positioned in relation to shaft (122). In the expanded configuration, end effector (200) is substantially flat and has an obliquely extending portion (210) that extends laterally from the longitudinal axis (LA) of catheter (120). By way of example only, end effector (200) may have a length ranging from approximately 20 mm to approximately 35 mm, or more particularly approximately 27 mm; and a width ranging from approximately 8 mm to approximately 15 mm, or more particularly approximately 11 mm. Alternatively, end effector (200) may have any other suitable dimensions.

End effector (200) includes a longitudinally extending base member (212), an array of obliquely extending members (214), a longitudinally extending parallel member (216), and a tail member (202) that couples end effector (200) with the distal end of shaft (122). Members (212, 214, 216) are in the form of strips in this example; and are formed by portions of flex circuit subassemblies (240, 260, 290) that are layered together as will be described in greater detail below. Base member (212) extends longitudinally along the longitudinal axis (LA) defined by shaft assembly (120). Parallel member (216) is parallel with the longitudinal axis (LA) defined by shaft assembly (120); yet is laterally offset from the longitudinal axis (LA) defined by shaft assembly (120). Obliquely extending members (214) extend obliquely from base member (212). In the present example, obliquely extending members (214) define oblique angles with base member (212); yet obliquely extending members (214) are parallel with each other.

In the present example, the oblique angle α defined between the proximal-most obliquely extending member (214) and base member (212) promotes deformation of end effector (200) when outer sheath (124) translates distally from the proximal position shown in FIG. 2B to the distal position shown in FIG. 2A, and more particularly in FIG. 3. In other words, the oblique angle α defined between the proximal-most obliquely extending member (214) and base member (212) may help to prevent the distal edge of outer sheath (124) from snagging on end effector (200); and may also promote end effector (200) achieving a collapsed state whereby end effector (200) fits within the interior of outer sheath (124). As the angle α approaches 90 degrees, this may make it more difficult for end effector (200) to be retracted into outer sheath (124) from the deployed state. As the angle α approaches 0 degrees, this may make it more difficult to suitably position electrodes (252) on end effector (200). Thus, it may be desirable to provide an angle that strikes the best balance between facilitating retraction of end effector (200) within outer sheath (124) while also allowing a suitable number and positioning of electrodes (252) on end effector (200). As shown in FIG. 3, the angle α s referenced with respect to the longitudinal axis and an edge of the adjacent member (248). By way of example only, each obliquely extending member (214 or 248) may define a respective angle with the longitudinal extending base member (212 or 242) or the longitudinal axis (LA) at any angle from 90 degrees to approximately 160 degrees; or more particularly at an oblique angle of approximately 140. In some other versions, obliquely extending members (214) define different respective angles α with base member (212). It should be understood that base member (212) of the present example extends along the longitudinal axis (LA), such that the angles α defined between obliquely extending members (214 in FIG. 2B or 248 in FIG. 3) and the longitudinal axis (LA) are the same as the angles defined between obliquely extending members (214 in FIG. 2B or 248 in FIG. 3) and base member (212 in FIG. 2B or 242 in FIG. 3).

In the present example, members (212, 214, 216) of end effector (200) define a parallelogram shape, with openings (220) formed therethrough. The parallelogram shape projects laterally from the longitudinal axis (LA) of catheter (120), such that end effector (200) is asymmetric about the longitudinal axis (LA) of catheter (120). Openings (220) may allow blood to flow through end effector (200). In addition, openings (220) may facilitate deformation of end effector (200) as end effector (200) transitions between the collapsed, contained state (FIG. 2A) and the expanded, deployed state (FIG. 2B). In the present example, end effector (200) is resiliently biased to achieve the expanded state when end effector (200) is deployed from outer sheath (124). Outer sheath (124) is thus configured to overcome the resilient bias of end effector (200) and thereby maintain end effector (200) in the collapsed state when end effector (200) is contained in outer sheath (124). In some other versions, end effector (200) is actively driven from the collapsed position to the expanded position, via one or more pull-wires, via one or more temperature-sensitive members, via one or more electrically actuated members, and/or via any other suitable actuation features.

Catheter (120) and end effector (200) may be in the state shown in FIG. 2A when catheter (120) is introduced into the body of the patient (PA); and during transit from the insertion site to the targeted cardiovascular region within the patient (PA). Once catheter (120) and end effector (200) reach the targeted cardiovascular region within the patient (PA), outer sheath (124) may be retracted proximally to expose end effector (200), thereby allowing end effector (200) to achieve the expanded, deployed state shown in FIG. 2B.

As best seen in FIG. 3, end effector (200) of the present example comprises three flex circuit subassemblies (240, 260, 290) that are layered together to form a laminate. By way of example only, flex circuit subassemblies (240, 260, 290) may be secured together via one or more adhesives and/or using any other suitable features or techniques.

Flex circuit subassembly (240) includes a substrate (242) that defines a base member portion (244), a parallel member portion (246), obliquely extending member portions (248), and a tail portion (250). When end effector (200) is in a fully assembled state, base member portion (244) of flex circuit subassembly (240) cooperates with corresponding base member portions (264, 294) of flex circuit subassemblies (260, 290) to form base member (212) of end effector (200). Similarly, parallel member portion (246) of flex circuit subassembly (240) cooperates with corresponding parallel member portions (266, 296) of flex circuit subassemblies (260, 290) to form parallel member (216) of end effector (200). Obliquely extending member portions (248) of flex circuit subassembly (240) cooperate with corresponding obliquely extending member portions (268, 298) of flex circuit subassemblies (260, 290) to form obliquely extending members (214) of end effector (200). Tail portion (250) of flex circuit subassembly (240) cooperates with corresponding tail portions (270, 299) of flex circuit subassemblies (260, 290) to form tail member (202) of end effector (200).

Flex circuit subassembly (240) of the present example further comprises a plurality of electrodes (252) that are arranged in pairs along obliquely extending member portions (248). In some versions, electrodes (252) are also positioned along base member portion (244) and/or parallel member portion (246). Each pair of electrodes (252) is operable to provide bipolar EP mapping by picking up electrocardiogram signals from tissue as is known in the art. By way of example only, the spacing of electrodes (252) in each pair of electrodes (252) may be arranged to conform to the arrangements shown and described in U.S. Pub. No. 2020/0297281, entitled "Electrode Configurations for Diagnosis of Arrhythmias," published September 24, 2020. Signals picked up by electrodes (252) may be communicated back through electrical conduits (not shown) in catheter (120) to console (12), which may process the signals to provide EP mapping to thereby identify locations of aberrant electrical activity within the cardiac anatomy. This may in turn allow the physician (PH) to identify the most appropriate regions of cardiac tissue to ablate (e.g., with RF energy, cryoablation, etc.), to thereby prevent or at least reduce the communication of aberrant electrical activity across the cardiac tissue. By way of example only, the number of electrodes (252) on flex circuit subassembly (240) may range from 40 to 60; or may be 50. Alternatively, flex circuit subassembly (240) may include any other suitable number of electrodes (252).

Substrate (242) may be formed of polyimide, polyether ether ketone, or any other suitable flex circuit substrate; with electrodes (252) and associated conductive traces being printed on substrate (242). In some versions, substrate (242) is formed of a resilient material such as nitinol to resiliently bias end effector (200) to the expanded configuration shown in FIG. 2 when end effector (200) is exposed relative to outer sheath (124). In some other versions, one or more resilient supports are secured to substrate (242) to provide such resilience. As another variation, substrate (242) may include a polymeric layer laid over a resilient (e.g., nitinol) layer.

Electrodes (252) may be formed of platinum, gold, or any other suitable material. Electrodes (252) may include various coatings, if desired. For instance, electrodes (252) may include a coating that is selected to improve the signal-to-noise ratio of signals from pairs of electrodes (252). Such coatings may include, but need not be limited to, iridium oxide (IrOx) coating, poly(3,4-ethylenedioxythiophene) (PEDOT) coating, Electrodeposited Iridium Oxide (EIROF) coating, Platinum Iridium (PtIr) coating, or any other suitable coating.

In some versions, electrodes (252) may be provided on substrate (242) as a thin film through a physical vapor deposition (PVD) process. By way of example only, such a PVD process may be carried out in accordance with at least some of the teachings of International Patent Pub. No. WO 2015/117908, entitled "Medical Device for Ablating Tissue Cells and System Comprising a Device of This Type," published August 13, 2015, at least some of the teachings of German Patent Pub. No. 102017130152, entitled "Method for Operating a Multi-Layer Structure," published January 3, 2019, or at least some of the teachings of U.S. Pat. No. 10,061,198, entitled "Method for Producing a Medical Device or a Device with Structure Elements, Method for Modifying the Surface of a Medical Device or of a Device with Structure Elements, Medical Device and Laminated Composite with a Substrate," published August 28, 2018. Other methods may also be employed to provide electrodes (252), conductive traces, or other circuit components on substrate (242), including but not limited to sputter deposition, chemical vapor deposition (CVD), thermal deposition, etc.

Tail portion (250) includes a plurality of termination pads (254) in the present example. Termination pads (254) are in electrical communication with electrodes (252) via corresponding traces (not shown) formed in flex circuit subassembly (240). Wires (not shown) are coupled with termination pads (254) and extend along the interior of catheter (120) to provide a path for electrical communication from electrodes (252) to console (12). In some other versions, a flex circuit extends along the length of catheter (120) to provide a path for electrical communication from electrodes (252) to console (12). While tail portion (250) is shown in a flat configuration in FIG. 3, tail portion (250) may be wrapped about the distal end of shaft (122) when end effector (200) is fully assembled to catheter (120).

Flex circuit subassembly (260) includes a substrate (262) that defines a base member portion (264), a parallel member portion (266), obliquely extending member portions (268), and a tail portion (260). When end effector (200) is in a fully assembled state, base member portion (264) of flex circuit subassembly (260) cooperates with corresponding base member portions (244, 294) of flex circuit subassemblies (240, 290) to form base member (212) of end effector (200). Similarly, parallel member portion (266) of flex circuit subassembly (260) cooperates with corresponding parallel member portions (246, 296) of flex circuit subassemblies (240, 290) to form parallel member (216) of end effector (200). Obliquely extending member portions (268) of flex circuit subassembly (260) cooperate with corresponding obliquely extending member portions (248, 298) of flex circuit subassemblies (240, 290) to form obliquely extending members (214) of end effector (200). Tail portion (270) of flex circuit subassembly (260) cooperates with corresponding tail portions (240, 299) of flex circuit subassemblies (240, 290) to form tail member (202) of end effector (200). While tail portion (270) is shown in a flat configuration in FIG. 3, tail portion (270) may be wrapped about the distal end of shaft (122) when end effector (200) is fully assembled to catheter (120).

Flex circuit subassembly (260) of the present example further comprises a plurality of position sensor traces (272, 276, 280) that form corresponding position sensor coils (274, 278, 282). Position sensor trace (272) extends along base member portion (264), the two distal-most obliquely extending member portions (268), parallel member portion (266), and tail portion (260). Position sensor trace (276) extends along base member portion (264), the two proximal-most obliquely extending member portions (268), parallel member portion (266), and tail portion (260). Position sensor trace (280) is positioned entirely on tail portion (270). Thus, position sensor coil (274) is positioned distal to position sensor coils (278, 282); position sensor coil (278) is positioned proximal to position sensor coil (274) and distal to position sensor coil (282); and position sensor coil (282) is positioned proximal to position sensor coils (274, 278).

Each position sensor coil (274, 278, 282) is operable to generate signals that are indicative of the position and orientation of the corresponding portion of end effector (200) within the patient (PA). Position sensor coils (274, 278, 282) generate electrical signals in response to the presence of an alternating electromagnetic field generated by field generators (20). Each position sensor coil (274, 278, 282) may be coupled with corresponding wire, a corresponding trace, or any other suitable corresponding electrical conduit along or otherwise through catheter (120), thereby enabling signals generated by position sensor coils (274, 278, 282) to be communicated back through electrical conduits (not shown) in catheter (120) to console (12). Console (12) may process the signals from position sensor coils (274, 278, 282) to identify the positions of the corresponding portions of end effector (200) within the patient (PA).

Other components and techniques that may be used to generate real-time position data associated with end effector (200) may include wireless triangulation, acoustic tracking, optical tracking, inertial tracking, and the like. In some versions, position sensor coils (274, 278, 282) may be omitted. In versions providing position sensor coils (274, 278, 282) or other position tracking features, the position data may be correlated with the EP mapping data picked up by electrodes (252) such that console (12) may store the locations of EP signals in conjunction with the precise anatomical locations where such EP signals were generated within the patient (PA).

Substrate (262) may be formed of polyimide, polyether ether ketone, or any other suitable flex circuit substrate; with position sensor traces (272, 276, 280) being printed on substrate (262). In some versions, substrate (262) is formed of a resilient material such as nitinol to resiliently bias end effector (200) to the expanded configuration shown in FIG. 2 when end effector (200) is exposed relative to outer sheath (124). In some other versions, one or more resilient supports are secured to substrate (262) to provide such resilience. As another variation, substrate (262) may include a polymeric layer laid over a resilient (e.g., nitinol) layer. Regardless of the material used to form substrate (262), sensor traces (272, 276, 280) may be provided on substrate (262) as a thin film through a physical vapor deposition (PVD) process, sputter deposition, chemical vapor deposition (CVD), thermal deposition, or any other suitable process.

Flex circuit subassembly (260) of the present example further comprises a temperature sensor (284). By way of example only, temperature sensor (284) may comprise a thermocouple and/or any other suitable kind of configuration. Temperature sensor (284) is positioned on parallel member portion (266). However, other versions may provide temperature sensor (284) on base member portion (264) and/or on one or more obliquely extending member portions (268). Temperature sensor (284) is configured to sense the temperature at the corresponding region of end effector (200). Temperature data from temperature sensor (284) may be communicated to console (12), which may factor such temperature into one or more control algorithms in any suitable fashion. By way of example only, temperature data from temperature sensor (284) may be utilized to further refine EP signal data from electrodes (252). In addition, or in the alternative, in versions where end effector (200) includes one or more ablation electrodes, temperature data from temperature sensor (284) may be utilized to regulate the delivery of power to such ablation electrodes, to thereby prevent overheating of end effector (200) and/or overheating of adjacent tissue. While just one temperature sensor (284) is shown in FIG. 3, other variations of end effector (200) may include more than one temperature sensor (284); or no temperature sensor (284) at all. While temperature sensor (284) is shown as being part of flex circuit subassembly (260) in FIG. 3, one or more temperature sensors (284) may be integrated into either or both of the other flex circuit subassemblies (240, 290), in addition to or as an alternative to being integrated into flex circuit subassembly (260).

Flex circuit subassembly (290) includes a substrate (292) that defines a base member portion (294), a parallel member portion (296), obliquely extending member portions (298), and a tail portion (299). When end effector (200) is in a fully assembled state, base member portion (294) of flex circuit subassembly (290) cooperates with corresponding base member portions (244, 264) of flex circuit subassemblies (240, 260) to form base member (212) of end effector (200). Similarly, parallel member portion (296) of flex circuit subassembly (290) cooperates with corresponding parallel member portions (246, 266) of flex circuit subassemblies (240, 260) to form parallel member (216) of end effector (200). Obliquely extending member portions (298) of flex circuit subassembly (290) cooperate with corresponding obliquely extending member portions (248, 268) of flex circuit subassemblies (240, 260) to form obliquely extending members (214) of end effector (200). Tail portion (299) of flex circuit subassembly (290) cooperates with corresponding tail portions (240, 270) of flex circuit subassemblies (240, 260) to form tail member (202) of end effector (200). While tail portion (299) is shown in a flat configuration in FIG. 3, tail portion (299) may be wrapped about the distal end of shaft (122) when end effector (200) is fully assembled to catheter (120).

Flex circuit subassembly (290) may be configured an operable just like flex circuit subassembly (240), but with electrodes and termination pads facing in an opposite direction to electrodes (252) and termination pads (254). In other words, the electrodes and termination pads of flex circuit assembly (290) may face downwardly in the view shown in FIG. 3. End effector (200) may thus present electrodes on opposing faces of end effector (200) when end effector (200) is in a fully assembled state.

In an example of a method where end effector (200) is used in the patient (PA), the physician (PH) may initially insert catheter (120) into a major vein or artery (e.g., the femoral artery) and then advance catheter (120) to position end effector (200) in the heart (H) of the patient (200). During this advancement of catheter (120), console (12) may track the position of catheter (120) in real time based on signals from position sensor coils (274, 278, 282) and render the real-time position on display (18), such that the physician (PH) may observe display (18) to determine the real-time position of catheter (120) (e.g., in relation to a preoperatively obtained image of the patient (PA)). Outer sheath (124) may be in the distal position (FIG. 2A) during the advancement of catheter (120) into the patient (PA).

Once the distal end of catheter (120) is suitably positioned, the physician (PH) may retract outer sheath (124) proximally to uncover end effector (200). End effector (200) may then reach the expanded state as shown in FIG. 2B. With end effector (200) in the expanded state, the physician (PH) may press end effector (200) against anatomical structures in the cardiac system of the patient (PA), such as the wall of the pulmonary vein, the wall of an atrium or other chamber of the heart (H), etc. In some procedures, the physician (PH) may apply end effector (200) against the tissue in a brushing motion, in a stamping motion, and/or using any other suitable technique(s). As end effector (200) engages these anatomical structures, electrodes (252) that are in contact with the tissue will pick up signals from the tissue, thereby enabling console (12) to perform EP mapping. Console (12) may correlate the signals from electrodes (252) with contemporaneous signals from position sensor coils (274, 278, 282) to thereby enable storage of the precise locations of aberrant electrical signals within the cardiac tissue of the patient (PA). Console (12) may thus enable mapping of aberrant electrical signal sites with respect to a preoperatively obtained image of the cardiac anatomy of the patient (PA). With such a map being established, the physician (PH) may then provide precisely targeted ablation (e.g., using end effector (200) or some other device) or some other kind of targeted therapy based on the map.

The flexibility of end effector (200) may allow end effector (200) to conform to the contours and other surface geometry of cardiac tissue when end effector (200) is pressed against cardiac tissue (e.g., within a chamber of the heart (H), within the pulmonary vein, etc.). The deformation of end effector (200) may promote full contact between electrodes (252) and cardiac tissue. Such contact may be further promoted by providing a substantial number of electrodes (252) on end effector (200). Having a substantial number of electrodes (252) may enable end effector (200) to provide high density EP mapping through all four chambers of the heart (H), within the pulmonary vein, etc., as several pairs of electrodes (252) may provide electrocardiogram signal sensing at multiple regions of cardiac tissue simultaneously.

In some scenarios, only one side of end effector (200) will be contacting tissue at any given moment of operation where end effector (200) is contacting tissue. In other words, either one or more pairs of electrodes (252) of only flex circuit subassembly (240) may be contacting tissue, without any electrodes (not shown) of flex circuit subassembly (290) contacting tissue; or one or more pairs of electrodes (not shown) of only flex circuit subassembly (290) may be contacting tissue, without any electrodes (252) of flex circuit subassembly (240) contacting tissue. The electrodes of end effector (200) that are not contacting tissue may nevertheless be contacting the blood that is circulating through the cardiovascular system of the patient (PA). Any electrodes of end effector (200) that are contacting blood may serve as reference electrodes, picking up reference potentials from the blood to reduce noise or far field signals, as is known in the art.

While only EP mapping electrodes (252) are shown on end effector (200) in the present example, other versions of end effector (200) may include one or more ablation electrodes in addition to, or in lieu of, including EP mapping electrodes (252). Such ablation electrodes may be used to apply RF energy to tissue that is in contact with the ablation electrodes, to thereby ablate the tissue. Each ablation electrode may be coupled with a corresponding trace or other electrical conduit of end effector (200), thereby enabling console (12) to communicate RF energy through electrical conduits (not shown) in catheter (120) to the traces or other conduits of end effector (200) to reach the ablation electrodes. Alternatively, the mapping electrodes (252) may be connected for both mapping or ablating in which the electrodes (252) may map (but not ablate while mapping) or ablate (but not mapping during the ablation).

As noted above, end effector (200) may tend to deform as end effector (200) is pressed against tissue during normal use. This deformation of end effector (200) may cause deformation of position sensor coils (274, 278). In some scenarios, the deformation of position sensor coils (274, 278) may affect the signals that are generated by position sensor coils (274, 278). To the extent that deformation of position sensor coils (274, 278) may compromise the accuracy of position readings through signals generated by position sensor coils (274, 278), it may be desirable to provide additional signal processing to account for such signal effects caused by deformation of position sensor coils (274, 278).

While catheter assembly (100) of the present example provides position sensing via position sensor coils (274, 278, 280), some versions may provide position sensing using other technologies in addition to, or in lieu of, providing position sensing via position sensor coils (274, 278, 280). By way of example only, alternative position sensing may include impedance measurement-based position sensing. Examples of such position sensing are described in U.S. Pat. No. 7,869,865, entitled "Current-Based Position Sensing," issued January 11, 2011, and U.S. Pat. No. 8,456,182, entitled "Current Localization Tracker," issued June 4, 2013, the disclosure of which is incorporated by reference herein, in its entirety. 2013. Alternatively, any other suitable position sensing technologies and techniques may be used.

### III. Example of End Effector with Alternative Flex Circuit Configuration

In end effector (200) described above, position sensor coils (274, 278, 280) and temperature sensor (284) are provided on a flex circuit subassembly (260) that is manufactured separately from flex circuit subassemblies (240, 290) that include electrodes (252). While flex circuit subassemblies (240, 260, 290) are ultimately joined together to form end effector (200), it may be desirable to integrate electrodes like electrodes (252), position sensor coils like position sensor coils (274, 278, 280), and a temperature sensor like temperature sensor (284) into the same flex circuit subassembly. In other words, it may be desirable to provide a single substrate that has a combination of electrodes like electrodes (252), position sensor coils like position sensor coils (274, 278, 280), and a temperature sensor like temperature sensor (284) on the same single substrate. Such an arrangement may provide efficiencies in manufacturing processing time and use of materials.

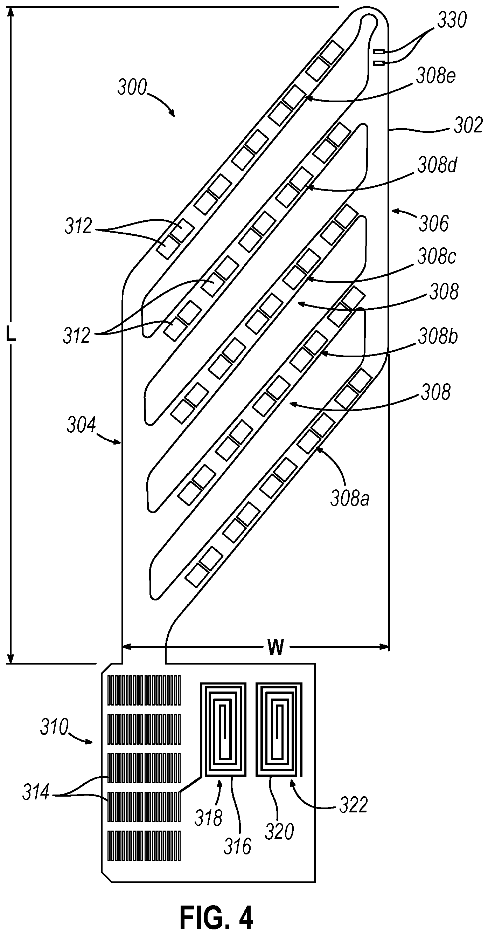

FIG. 4 shows an example of an end effector (300) that includes electrodes (312), position sensor coils (318, 322), and temperature sensors (220) all on the same single substrate (302). End effector (300) may be otherwise configured and operable similar to end effector (200); and may be positioned at the distal end of catheter (120) in place of end effector (200). Substrate (302) of end effector (300) defines a base member portion (304), a parallel member portion (306), obliquely extending member portions (308), and a tail portion (310).

Electrodes (312) are arranged in pairs along obliquely extending member portions (308). In some versions, electrodes (312) are also positioned along base member portion (304) and/or parallel member portion (306). Each pair of electrodes (312) is operable to provide bipolar EP mapping by picking up electrocardiogram signals from tissue as described above. Signals picked up by electrodes (312) may be communicated back through electrical conduits (not shown) in catheter (120) to console (12), which may process the signals to provide EP mapping to thereby identify locations of aberrant electrical activity within the cardiac anatomy as described above. Electrodes (312) may be configured, operable, and manufactured like electrodes (252) described above.

In one example, the electrode (312) is approximately 0.8 mm by 0.5 mm in a pair. In the example of FIG. 4, each pair of electrodes (312) is spaced apart from the adjacent pair at approximately 2.4 mm for a of total ten electrodes (312) per oblique arm (308 a - 308e) for a total of fifty electrodes (312). End effector (300) has a coverage area (A) of length (L) of approximately 27 mm and a width (W) of approximately 11mm so that the number of electrodes (312) over the area (A) is approximately 17 electrodes (312) per squared centimeter. While the drawings show electrodes (312) as being on one surface of each arm (308a - 308e) (the surface visible in the drawings), it should be understood that a similar number of electrodes (312) in the same arrangement can be provided on the opposite side (i.e., on the opposite surface or under the top surface) of each arm (308a - 308e). The placement of electrodes (312) on opposite sides of the arms (308a - 308e) allows for electrodes (312) on one side to contact tissues (and record signals emanating from tissues) while electrodes (312) not in contact with tissues (on the opposite side of the same arm) can be used to collect far-field signals for noise reduction of the tissue generated signals collected from electrodes (312) in direct physical contact with tissues.

Temperature sensors (330) are provided on parallel member portion (306) in the present example, though temperature sensors (330) may alternatively be positioned elsewhere on substrate (302). By way of example only, each temperature sensor (330) may comprise a thermocouple and/or any other suitable kind of configuration. Each temperature sensor (330) is configured to sense the temperature at the corresponding region of end effector (300). Temperature data from temperature sensors (330) may be communicated to console (12), which may factor such temperature into one or more control algorithms in any suitable fashion, as described above in the context of temperature sensor (284).

Tail portion (310) includes a plurality of termination pads (314) in the present example. Termination pads (314) are in electrical communication with electrodes (312) via corresponding traces (not shown) formed in the flex circuit of end effector (300). Wires (not shown) are coupled with termination pads (314) and extend along the interior of catheter (120) to provide a path for electrical communication from electrodes (312) to console (12). In some other versions, a flex circuit extends along the length of catheter (120) to provide a path for electrical communication from electrodes (312) to console (12). While tail portion (310) is shown in a flat configuration in FIG. 4, tail portion (310) may be wrapped about the distal end of shaft (122) when end effector (300) is fully assembled to catheter (120).

Position sensor coils (318, 322) of the present example are formed by position sensor traces (316, 320), which are located on tail portion (310) in the present example. In some versions, position sensor traces (316, 320) are positioned on tail portion (310) such that position coils (318, 322) will be positioned about respective coil axes that are perpendicular to each other when tail portion (310) is wrapped about the distal end of catheter (120). While position sensor coils (318, 322) are only located on tail portion (310) in this example, other variations may provide position sensor coils along other portions (304, 306, 308) of substrate (302), in addition to or in lieu of providing position sensor coils (318, 322) on tail portion (310).

Each position sensor coil (318, 322) is operable to generate signals that are indicative of the position and orientation of at least the proximal end of end effector (300) within the patient (PA). Position sensor coils (318, 322) generate electrical signals in response to the presence of an alternating electromagnetic field generated by field generators (20). Each position sensor coil (318, 322) may be coupled with corresponding wire, a corresponding trace, or any other suitable corresponding electrical conduit along or otherwise through catheter (120), thereby enabling signals generated by position sensor coils (318, 322) to be communicated back through electrical conduits (not shown) in catheter (120) to console (12). Console (12) may process the signals from position sensor coils (318, 322) to identify the position of at least the proximal end of end effector (300) within the patient (PA). Position sensor coils (318, 322) may be configured, operable, and manufactured like position sensor coils (274, 278, 282) described above.

Substrate (302) may be formed of polyimide, polyether ether ketone, or any other suitable flex circuit substrate; with electrodes (312) and associated conductive traces being printed on substrate (302). In some versions, substrate (302) is formed of a resilient material such as nitinol to resiliently bias end effector (200) to the expanded configuration shown in FIG. 4 when end effector (300) is exposed relative to outer sheath (124). In some other versions, one or more resilient supports are secured to substrate (302) to provide such resilience. As another variation, substrate (302) may include a polymeric layer laid over a resilient (e.g., nitinol) layer.

While not shown, the opposite side of end effector (300) may also have a plurality of electrodes (312), such that electrodes (312) may contact tissue regardless of which side of end effector (300) is facing the tissue. Electrodes (312) on the non-tissue-contacting side of end effector (300) may pick up reference signals from the blood of the patient (PA), as described above.

### IV. Example of End Effector with Deflectable Leaves

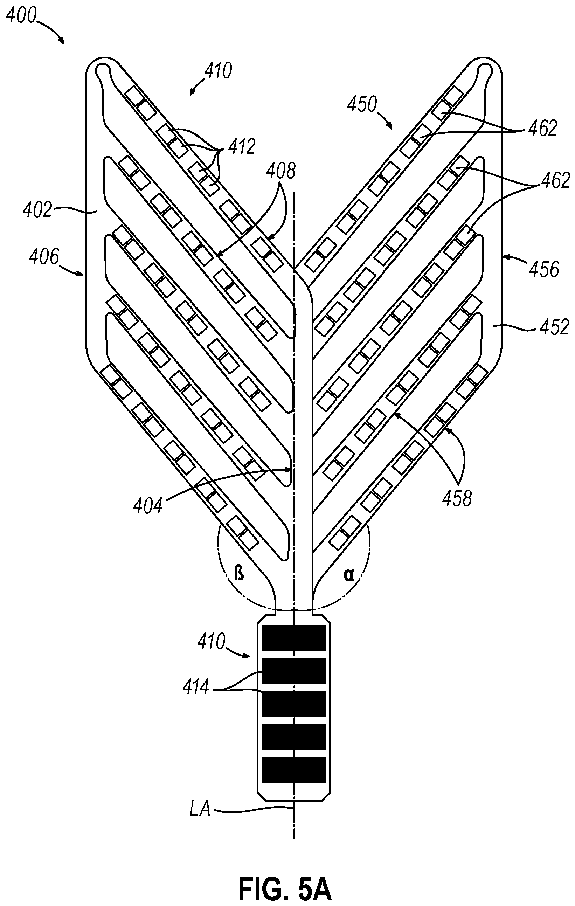

During an EP mapping procedure, it may be desirable to maximize the number of electrodes in contact with tissue at any given moment during the procedure. The exposed surface area of each end effector (200, 300) described above may limit how many electrodes (252, 312) may be integrated into end effector (200, 300). It may therefore be desirable to effectively increase the surface area of end effector (200, 300) to thereby increase the number of electrodes (252, 312) without adversely affecting other functional aspects of end effector (200, 300). To that end, FIGS. 5A-5B show an example of an alternative end effector (400) that includes two flex circuit leaves (410, 450). End effector (400) may be otherwise configured and operable similar to end effector (200); and may be positioned at the distal end of catheter (120) in place of end effector (200).

Flex circuit leaf (410) of the present example is configured and operable just like end effector (200) described above. Flex circuit leaf (410) thus includes a substrate (402) that defines a base member portion (404), a parallel member portion (406), obliquely extending member portions (408), and a tail portion (410). Electrodes (412) are positioned along obliquely extending member portions (408). Electrodes (412) may be configured and operable just like electrodes (252). Termination pads (414) are positioned along tail portion (410). Termination pads (414) may be configured and operable just like termination pads (254). While not shown in FIGS. 5A-5B, flex circuit leaf (410) may also include underlying flex circuit subassemblies that are configured and operable like flex circuit subassemblies (260, 290).

Flex circuit leaf (450) of the present example is also configured and operable just like end effector (200) described above. Flex circuit leaf (450) thus includes a substrate (452) that defines a base member portion (454), a parallel member portion (456), obliquely extending member portions (458), and a tail portion (not shown). Electrodes (462) are positioned along obliquely extending member portions (458). Electrodes (462) may be configured and operable just like electrodes (252). While not shown in FIGS. 5A-5B, flex circuit leaf (450) may also include underlying flex circuit subassemblies that are configured and operable like flex circuit subassemblies (260, 290).

Flex circuit leaves (410, 450) are positioned and oriented such that flex circuit leaf (450) mirrors flex circuit leaf (410). Flex circuit leaf (410) may be oriented at an oblique angle α relative to a central longitudinal axis (LA); while flex circuit leaf (450) may be oriented at an oblique angle β relative to the central longitudinal axis (LA). β may be equal to α or different from α in order to affect the sequence of folding. Where the two oblique angles α, β are equal, both leaves (410, 450) may tend to fold at approximately the same time as leaves (410, 450) are pulled back into outer sheath (124) (or as outer sheath (124) is advanced distally with leaves (410, 450) held stationary). In versions where the two oblique angles α, β are equal, leaves (410, 450) may tend to fold sequentially as leaves (410, 450) are pulled back into outer sheath (124) (or as outer sheath (124) is advanced distally with leaves (410, 450) held stationary).

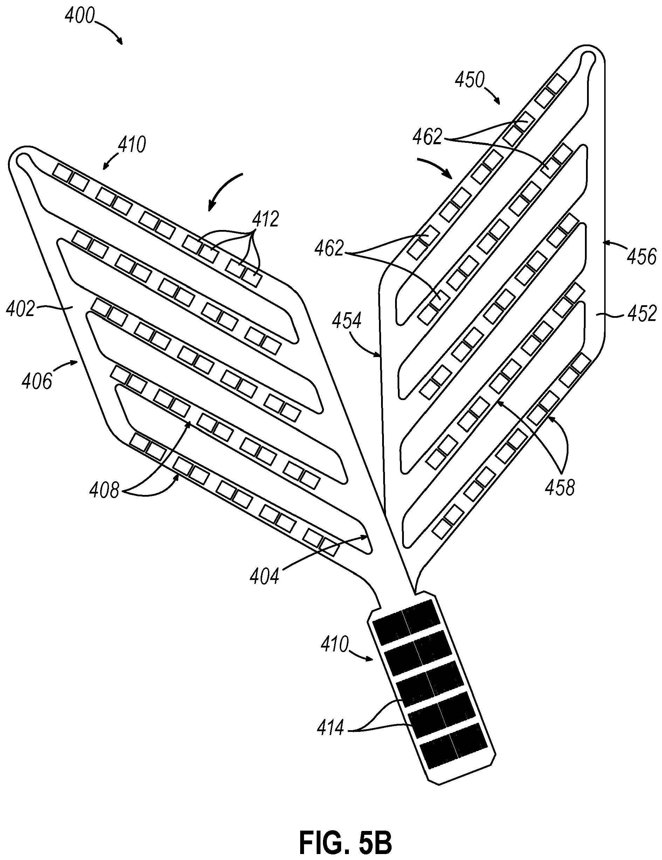

When flex circuit leaves (410, 450) are in a default deployed state as shown in FIG. 5A, base member portions (404, 454) overlap each other. However, flex circuit leaves (410, 450) are configured to deflect away from each other, as shown in FIG. 5B. Such deflection may occur as end effector (400) is pressed against tissue in a brushing motion, stamping motion, or other motion. Movability and deformation of flex circuit leaves (410, 450) relative to each other may promote enhanced engagement between electrodes (412, 462) of end effector (400) and tissue surfaces. Such enhanced engagement between electrodes (412, 462) of end effector (400) and tissue surfaces may allow end effector (400) to perform EP mapping in a faster and more efficient manner. Moreover, by having the proximal-most obliquely extending member portions (408, 458) being obliquely oriented as described above, end effector (400) may still be retracted into outer sheath (124) with relative ease; and without causing damage to end effector (400).

In the present example, flex circuit leaves (410, 450) are only fixedly joined together at tail portion (410), which is where flex circuit leaves (410, 450) are secured to the distal end of catheter (120). In some other versions, flex circuit leaves (410, 450) are also fixedly joined together along base member portions (404, 454). Alternatively, flex circuit leaves (410, 450) may have any other suitable relationships with each other.

While not shown, the opposite side of end effector (400) may also have a plurality of electrodes (412, 462), such that electrodes (412, 462) may contact tissue regardless of which side of end effector (400) is facing the tissue. Electrodes (412, 462) on the non-tissue-contacting side of end effector (400) may pick up reference signals from the blood of the patient (PA), as described above.

### V. Example of End Effector with Symmetric Unitary Body

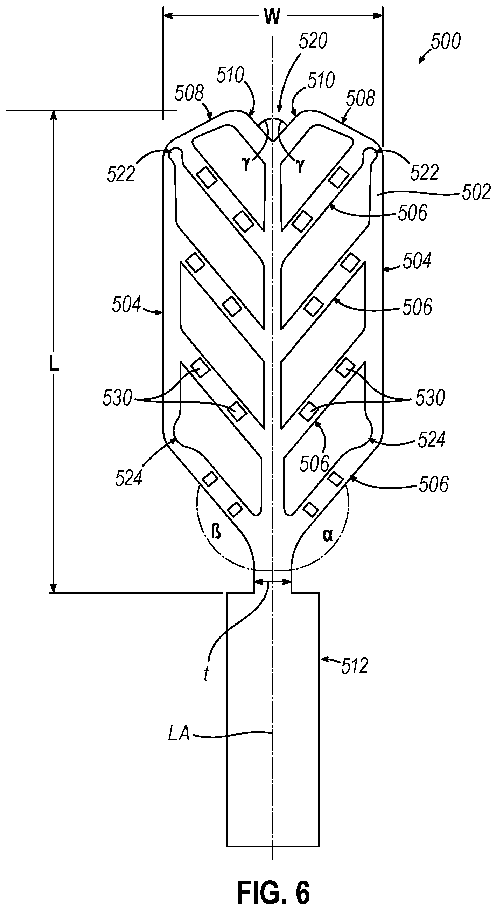

In some instances, it may be desirable to provide an EP mapping end effector that is symmetric about a longitudinal axis. It may be further desirable for such an end effector to include features that promote collapsing of the end effector from a deployed expanded state to a compressed or collapsed state within a sheath like outer sheath (124). To that end, FIG. 6 depicts an example of an end effector (500) that is symmetric about a longitudinal axis (LA) and that includes features that promote collapsing of end effector (500) from a deployed expanded state to a compressed or collapsed state within a sheath like outer sheath (124). End effector (500) may be secured to the distal end of shaft (122) in place of end effector (200). In the example of FIG. 6, end effector (500) may have a length (L) of approximately 27 mm and a width (W) of approximately 10 mm with a stem having a thickness "t" of approximately 1.4 mm. Except as otherwise described below, end effector (500) may be configured and operable like end effector (500).

End effector (500) of the present example includes a substrate (502) defining three longitudinally extending portions (504), four obliquely extending portions (506), two distal portions (508), two distal portions (510), and a tail portion (512). Obliquely extending portions (506) extend from a central longitudinally extending portion (504) to the outer longitudinally extending portions (504). In the present example, each obliquely extending portion (506) extends obliquely relative to the longitudinal axis. By way of example only, each obliquely extending portion (506) may define a respective angle α with the longitudinal axis (LA) at any angle from approximately 90 degrees to approximately 170 degrees; or more particularly at an oblique angle of approximately 140. In some other versions, obliquely extending portions (506) define different respective angles with the longitudinal axis (LA). For instance, one of the proximal-most obliquely extending portions (506) may be oriented at an oblique angle α while another one of the proximal-most obliquely extending portions (506) may be oriented at an oblique angle β that may be equal to α or different from α in order to affect the sequence of folding. Where the two oblique angles α, β are equal, both proximal-most obliquely extending portions (506) may tend to fold at approximately the same time as proximal-most obliquely extending portions (506) are pulled back into outer sheath (124) (or as outer sheath (124) is advanced distally with proximal-most obliquely extending portions (506) held stationary). In versions where the two oblique angles α, β are equal, the two proximal-most obliquely extending portions (506) may tend to fold sequentially as proximal-most obliquely extending portions (506) are pulled back into outer sheath (124) (or as outer sheath (124) is advanced distally with proximal-most obliquely extending portions (506) held stationary). It should be understood that central longitudinally extending portion (504) of the present example extends along the longitudinal axis (LA), such that the angles defined between obliquely extending portions (506) and the longitudinal axis (LA) are the same as the angles defined between obliquely extending portions (506) and central longitudinally extending portion (504).

A plurality of electrodes (530) are arranged in pairs along respective obliquely extending portions (506). Electrodes (530) and substrate (502) thus form a flex circuit. Electrodes (530) may be configured and operable just like electrodes (252). Substrate (502) may be formed of polyimide, polyether ether ketone, or any other suitable flex circuit substrate; with electrodes (530) and associated conductive traces being printed on substrate (502). In some versions, substrate (502) is formed of a resilient material such as nitinol to resiliently bias end effector (500) to the expanded configuration shown in FIG. 6 when end effector (500) is exposed relative to outer sheath (124). In some other versions, one or more resilient supports are secured to substrate (502) to provide such resilience. As another variation, substrate (502) may include a polymeric layer laid over a resilient (e.g., nitinol) layer.

While not shown, the opposite side of end effector (500) may also have a plurality of electrodes (530), such that electrodes (530) may contact tissue regardless of which side of end effector (500) is facing the tissue. Electrodes (530) on the non-tissue-contacting side of end effector (500) may pick up reference signals from the blood of the patient (PA), as described above.

Tail portion (512) may be wrapped about the exterior of the distal end of shaft (122) to thereby secure end effector (500) to shaft (122). Tail portion (512) may include termination pads similar to termination pads (254) described above. End effector (500) may also include one or more position sensor coils like sensor coils (274, 278, 282), one or more temperature sensors like temperature sensor (284), and/or any other suitable features.

End effector (500) of the present example further includes a plurality of recesses (520, 522, 524) that are specially positioned and configured to promote collapse of end effector (500) into outer sheath (124) as end effector (500) transitions from a deployed, expanded state (similar to what is shown in FIG. 2B) to a contained, compressed state (similar to what is shown in FIG. 2B). By promoting such collapse of end effector (500), recesses (520, 522, 524) may reduce the risk of end effector (500) snagging on the distal edge of outer sheath (124) during the transition, may reduce the risk of end effector (500) becoming damaged during or after the transition, and/or may provide other results. Recess (520) is formed between distal portions (510), which converge proximally toward the longitudinal axis (LA); while distal portions (508) are angled distally toward the longitudinal axis (LA). Recess (520) may provide a predefined buckling region for the distal end of end effector (500), such that end effector (500) may tend to buckle at recess (520) as end effector (500) transitions from a deployed, expanded state to a contained, compressed state. Distal portions (510) are obliquely oriented relative to the longitudinal axis (LA) to define recess (520). By way of example only, each distal portion (510) may define a respective angle γ with the longitudinal axis (LA) at any angle γ from approximately 90 degrees to approximately 170 degrees; or more particularly at an oblique angle γ of approximately 140.

Recesses (522) are formed by rounded edges at the interior regions of the intersections of the distal-most obliquely extending portions (506) and outer longitudinally extending portions (504). Recesses (522) may provide additional predefined bucking regions for the distal end of end effector (500), such that end effector (500) may tend to buckle at recesses (522) as end effector (500) transitions from a deployed, expanded state to a contained, compressed state. Recesses (524) are formed by rounded edges at the interior regions of the intersections of the proximal-most obliquely extending portions (506) and outer longitudinally extending portions (504). Recesses (524) may provide predefined bucking regions for the proximal end of end effector (500), such that end effector (500) may tend to buckle at recesses (524) as end effector (500) transitions from a deployed, expanded state to a contained, compressed state. Taken together, recesses (520, 522, 524) may all ensure that end effector (500) collapses in repeatable, predictable fashion, without end effector (500) being damaged by the collapse, as end effector (500) transitions from a deployed, expanded state to a contained, compressed state.

### VII. Miscellaneous

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

## Claims

1. An apparatus comprising:
(a) a catheter shaft assembly (100) having a proximal end and a distal end, the catheter shaft assembly defining a longitudinal axis (LA); and
(b) an end effector (200) associated with the distal end of the catheter shaft assembly, the end effector comprising a first flex circuit assembly, the first flex circuit assembly comprising:
(i) a base member (212) extending distally from the distal end of the catheter shaft assembly along the longitudinal axis,
(ii) a plurality of obliquely extending members (214) extending obliquely and distally from the base member, the obliquely extending members being configured to transition between a first configuration and a second configuration, the obliquely extending members being configured to fit within an outer sheath in the first configuration, the obliquely extending members being configured to expand outwardly away from the longitudinal axis in the second configuration when exposed distally relative to the distal end of the outer sheath,
(iii) a plurality of electrodes (252) positioned on at least some of the obliquely extending members, the electrodes being positioned to contact one or both of tissue or blood, and
(iv) an offset member (216), the offset member being laterally offset from the base member, the obliquely extending members extending between the base member to the offset member, the offset member being oriented parallel with the base member.

2. The apparatus of claim 1, the offset member, the base member, and two of the obliquely extending members together defining (i) a parallelogram shape or (ii) a plurality of openings (220).

3. The apparatus of any preceding claim, the obliquely extending members being obliquely oriented relative to the longitudinal axis.

4. The apparatus of any preceding claim, the end effector further comprising one or more position sensors (274, 278, 282) and plurality of electrodes disposed on opposite planar surfaces of each oblique members, preferably the one or more position sensors being part of the first flex circuit assembly.

5. The apparatus of any preceding claim, the end effector further comprising one or more temperature sensors (284).

6. The apparatus of any preceding claim, the end effector further comprising a resilient body, the resilient body being configured to resiliently bias the obliquely extending members to expand outwardly away from the longitudinal axis in the second configuration when exposed distally relative to the distal end of the outer sheath.

7. The apparatus of any preceding claim, the first flex circuit assembly further comprising:
(i) a first flex circuit subassembly (240), the first flex circuit subassembly including:
(A) a first flexible substrate, and
(B) at least some electrodes of the plurality of electrodes positioned along the first flexible substrate, and
(ii) a second flex circuit subassembly (260), the second flex circuit subassembly including:
(A) a second flexible substrate, the second flexible substrate being secured in apposition with the first flexible substrate to form a multi-layer laminate, and
(B) one or more electrical features configured to generate signals, the one or more electrical features being positioned along the second flexible substrate.

8. The apparatus of claim 7, the first flex circuit assembly further comprising a third flex circuit subassembly, the third flex circuit subassembly (290) including:
(A) a third flexible substrate, the third flexible substrate being secured in apposition with the second flexible substrate to form a multi-layer laminate, with the second flexible substrate being interposed between the first flexible substrate and the third flexible substrate, and
(B) at least some additional electrodes of the plurality of electrodes, the at least some additional electrodes being positioned along the third flexible substrate.

9. The apparatus of any preceding claim, the end effector further comprising a second flex circuit assembly (450), the second flex circuit assembly comprising:
(i) a plurality of obliquely extending members (458), the obliquely extending members of the second flex circuit assembly being configured to transition between a first configuration and a second configuration, the obliquely extending members of the second flex circuit assembly being configured to fit within an outer sheath in the first configuration, the obliquely extending members of the second flex circuit assembly being configured to expand outwardly away from the longitudinal axis in the second configuration when exposed distally relative to the distal end of the outer sheath, and
(ii) a plurality of electrodes (462) positioned on at least some of the obliquely extending members of the second flex circuit assembly, the electrodes of the second flex circuit assembly being positioned to contact one or both of tissue or blood.

10. The apparatus of any preceding claim, the base member and the plurality of obliquely extending members being configured to extend along a flat plane when exposed distally relative to the distal end of the outer sheath.

11. The apparatus according to claim 1, wherein the first base member and the first plurality of obliquely extending members are configured to extend along a first flat plane when exposed distally relative to the distal end of the outer sheath, wherein the apparatus further comprises:
(i) a second flex circuit assembly (450), the second flex circuit assembly comprising:
(A) a second base member (454) extending distally from the distal end of the catheter shaft assembly,
(B) a second plurality of obliquely extending members (458) extending obliquely from the base member, second plurality of obliquely extending members being configured to expand outwardly away from the longitudinal axis in the second configuration when exposed distally relative to the distal end of the outer sheath, the second base member and the second plurality of obliquely extending members being configured to extend along a second flat plane when exposed distally relative to the distal end of the outer sheath, and
(C) a second plurality of electrodes (462) positioned on the second plurality of obliquely extending members, the second plurality of electrodes being positioned to contact one or both of tissue or blood.

12. The apparatus according to any preceding claim, the first flex circuit assembly further comprising a plurality of predefined buckling regions (520, 522, 524), the predefined buckling regions being configured to buckle in response to retraction of the end effector into the outer sheath.

## Patentansprüche

1. Einrichtung, umfassend:
(a) eine Katheterschaftanordnung (100), die ein proximales Ende und ein distales Ende aufweist, wobei die Katheterschaftanordnung eine Längsachse (LA) definiert; und
(b) einen Endeffektor (200), der mit dem distalen Ende der Katheterschaftanordnung verknüpft ist, der Endeffektor umfassend eine erste Flexschaltungsanordnung, die erste Flexschaltungsanordnung umfassend:
(i) ein Basiselement (212), das sich distal von dem distalen Ende der Katheterschaftanordnung entlang der Längsachse erstreckt,
(ii) eine Vielzahl von sich schräg erstreckenden Elementen (214), die sich schräg und distal von dem Basiselement erstrecken,
wobei die sich schräg erstreckenden Elemente konfiguriert sind, um zwischen einer ersten Konfiguration und einer zweiten Konfiguration zu wechseln, wobei die sich schräg erstreckenden Elemente konfiguriert sind, um in der ersten Konfiguration innerhalb einer Außenhülle zu passen, wobei die sich schräg erstreckenden Elemente konfiguriert sind, um sich in der zweiten Konfiguration nach außen weg von der Längsachse auszudehnen, wenn sie distal relativ zu dem distalen Ende der Außenhülle freigelegt werden,
(iii) eine Vielzahl von Elektroden (252), die an mindestens einigen der sich schräg erstreckenden Elemente positioniert sind, wobei die Elektroden positioniert sind, um eines oder beides von Gewebe oder Blut berühren, und
(iv) ein Versatzelement (216), wobei das Versatzelement von dem Basiselement seitlich versetzt ist, wobei sich die schräg verlaufenden Elemente zwischen dem Basiselement und dem Versatzelement erstrecken, wobei das Versatzelement parallel zu dem Basiselement ausgerichtet ist.

2. Einrichtung nach Anspruch 1, wobei das Versatzelement, das Basiselement und zwei der sich schräg erstreckenden Elemente zusammen (i) eine Parallelogrammform oder (ii) eine Vielzahl von Öffnungen (220) definieren.

3. Einrichtung nach einem der vorstehenden Ansprüche, wobei die sich schräg erstreckenden Elemente relativ zu der Längsachse schräg ausgerichtet sind.

4. Einrichtung nach einem der vorstehenden Ansprüche, der Endeffektor ferner umfassend einen oder mehrere Positionssensoren (274, 278, 282) und eine Vielzahl von Elektroden, die auf gegenüberliegenden ebenen Oberflächen jedes schrägen Elements angeordnet sind, wobei der eine oder die mehreren Positionssensoren vorzugsweise Teil der ersten Flexschaltungsanordnung sind.

5. Einrichtung nach einem der vorstehenden Ansprüche, der Endeffektor ferner umfassend einen oder mehrere Temperatursensoren (284).

6. Einrichtung nach einem der vorstehenden Ansprüche, der Endeffektor ferner umfassend einen elastischen Körper, wobei der elastische Körper konfiguriert ist, um die sich schräg erstreckenden Elemente elastisch vorzuspannen, um sich in der zweiten Konfiguration nach außen weg von der Längsachse auszudehnen, wenn sie distal relativ zu dem distalen Ende der Außenhülle freigelegt werden.

7. Einrichtung nach einem der vorstehenden Ansprüche, die erste Flexschaltungsanordnung ferner umfassend:
(i) eine erste Flexschaltungsunteranordnung (240), wobei die erste Flexschaltungsunteranordnung einschließt:
(A) ein erstes flexibles Substrat, und
(B) mindestens einige Elektroden der Vielzahl von Elektroden, die entlang des ersten flexiblen Substrats positioniert sind, und
(ii) eine zweite Flexschaltungsunteranordnung (260), wobei die zweite Flexschaltungsunteranordnung einschließt:
(A) ein zweites flexibles Substrat, wobei das zweite flexible Substrat in Anlagerung an dem ersten flexiblen Substrat befestigt ist, um ein mehrschichtiges Laminat auszubilden, und
(B) ein oder mehrere elektrische Merkmale, die konfiguriert sind, um Signale zu erzeugen, wobei das eine oder die mehreren elektrischen Merkmale entlang des zweiten flexiblen Substrats positioniert sind.

8. Einrichtung nach Anspruch 7, die erste Flexschaltungsanordnung ferner umfassend eine dritte Flexschaltungsunteranordnung, wobei die dritte Flexschaltungsunteranordnung (290) einschließt:
(A) ein drittes flexibles Substrat, wobei das dritte flexible Substrat in Anlagerung an dem zweiten flexiblen Substrat befestigt ist, um ein mehrschichtiges Laminat auszubilden, wobei das zweite flexible Substrat zwischen dem ersten flexiblen Substrat und dem dritten flexiblen Substrat eingeschoben ist, und
(B) mindestens einige zusätzliche Elektroden der Vielzahl von Elektroden, wobei die mindestens einigen zusätzlichen Elektroden entlang des dritten flexiblen Substrats positioniert sind.

9. Einrichtung nach einem der vorstehenden Ansprüche, der Endeffektor ferner umfassend eine zweite Flexschaltungsanordnung (450), die zweite Flexschaltungsanordnung umfassend:
(i) eine Vielzahl von sich schräg erstreckenden Elementen (458), wobei die sich schräg erstreckenden Elemente der zweiten Flexschaltungsanordnung konfiguriert sind, um zwischen einer ersten Konfiguration und einer zweiten Konfiguration zu wechseln, wobei die sich schräg erstreckenden Elemente der zweiten Flexschaltungsanordnung konfiguriert sind, um in der ersten Konfiguration innerhalb einer Außenhülle zu passen, wobei die sich schräg erstreckenden Elemente der zweiten Flexschaltungsanordnung konfiguriert sind, um sich in der zweiten Konfiguration nach außen weg von der Längsachse auszudehnen, wenn sie distal relativ zu dem distalen Ende der Außenhülle freigelegt werden, und
(ii) eine Vielzahl von Elektroden (462), die an mindestens einigen der sich schräg erstreckenden Elemente der zweiten Flexschaltungsanordnung positioniert sind, wobei die Elektroden der zweiten Flexschaltungsanordnung positioniert sind, um eines oder beides von Gewebe oder Blut zu berühren.

10. Einrichtung nach einem der vorstehenden Ansprüche, wobei das Basiselement und die Vielzahl von sich schräg erstreckenden Elementen konfiguriert sind, um sich entlang einer flachen Ebene zu erstrecken, wenn sie distal relativ zu dem distalen Ende der Außenhülle freigelegt werden.

11. Einrichtung nach Anspruch 1, wobei das erste Basiselement und die erste Vielzahl von sich schräg erstreckenden Elementen konfiguriert sind, um sich entlang einer ersten flachen Ebene zu erstrecken, wenn sie distal relativ zu dem distalen Ende der Außenhülle freigelegt werden, wobei die Einrichtung ferner umfasst:
(i) eine zweite Flexschaltungsanordnung (450), die zweite Flexschaltungsanordnung umfassend:
(A) ein zweites Basiselement (454), das sich distal von dem distalen Ende der Katheterschaftanordnung erstreckt,
(B) eine zweite Vielzahl von sich schräg erstreckenden Elementen (458), die sich schräg von dem Basiselement erstrecken, wobei die zweite Vielzahl von sich schräg erstreckenden Elementen konfiguriert sind, um sich in der zweiten Konfiguration nach außen weg von der Längsachse auszudehnen, wenn sie distal relativ zu dem distalen Ende der Außenhülle freigelegt werden, wobei das zweite Basiselement und die zweite Vielzahl von sich schräg erstreckenden Elementen konfiguriert sind, um sich entlang einer zweiten flachen Ebene zu erstrecken, wenn sie distal relativ zu dem distalen Ende der Außenhülle freigelegt werden, und
(C) eine zweite Vielzahl von Elektroden (462), die an der zweiten Vielzahl von sich schräg erstreckenden Elementen positioniert sind, wobei die zweite Vielzahl von Elektroden positioniert sind, um eines oder beides von Gewebe oder Blut zu berühren.

12. Einrichtung nach einem der vorstehenden Ansprüche, die erste Flexschaltungsanordnung ferner umfassend eine Vielzahl von vordefinierten Biegebereichen (520, 522, 524), wobei die vordefinierten Biegebereiche konfiguriert sind, um sich als Reaktion auf ein Zurückziehen des Endeffektors in die Außenhülle zu verbiegen.

## Revendications

1. Appareil comprenant :
(a) un ensemble tige de cathéter (100) ayant une extrémité proximale et une extrémité distale, l'ensemble tige de cathéter définissant un axe longitudinal (LA) ; et
(b) un effecteur terminal (200) associé à l'extrémité distale de l'ensemble tige de cathéter, l'effecteur terminal comprenant un premier ensemble circuit flexible, le premier ensemble circuit flexible comprenant :
(i) un élément de base (212) s'étendant de manière distale à partir de l'extrémité distale de l'ensemble tige de cathéter le long de l'axe longitudinal,
(ii) une pluralité d'éléments s'étendant de manière oblique (214) s'étendant de manière oblique et de manière distale à partir de l'élément de base,
les éléments s'étendant de manière oblique étant configurés pour effectuer une transition entre une première configuration et une seconde configuration, les éléments s'étendant de manière oblique étant configurés pour s'ajuster au sein d'une gaine externe dans la première configuration, les éléments s'étendant de manière oblique étant configurés pour se déployer vers l'extérieur à l'écart de l'axe longitudinal dans la seconde configuration lorsqu'ils sont exposés de manière distale relativement à l'extrémité distale de la gaine externe,
(iii) une pluralité d'électrodes (252) positionnées sur au moins certains des éléments s'étendant de manière oblique, les électrodes étant positionnées pour entrer en contact avec l'un ou les deux parmi les tissus ou le sang, et
(iv) un élément de décalage (216), l'élément de décalage étant décalé de manière latérale par rapport à l'élément de base, les éléments s'étendant de manière oblique s'étendant entre l'élément de base et l'élément de décalage, l'élément de décalage étant orienté parallèle à l'élément de base.

2. Appareil selon la revendication 1, l'élément de décalage, l'élément de base, et deux des éléments s'étendant de manière oblique définissant ensemble (i) une forme de parallélogramme ou (ii) une pluralité d'ouvertures (220).

3. Appareil selon l'une quelconque revendication précédente, les éléments s'étendant de manière oblique étant orientés de manière oblique relativement à l'axe longitudinal.

4. Appareil selon l'une quelconque revendication précédente, l'effecteur terminal comprenant en outre un ou plusieurs capteurs de position (274, 278, 282) et une pluralité d'électrodes disposées sur des surfaces planes opposées de chaque élément oblique, de préférence le ou les capteurs de position faisant partie du premier ensemble circuit flexible.

5. Appareil selon l'une quelconque revendication précédente, l'effecteur terminal comprenant en outre un ou plusieurs capteurs de température (284).

6. Appareil selon l'une quelconque revendication précédente, l'effecteur terminal comprenant en outre un corps élastique, le corps élastique étant configuré pour solliciter élastiquement les éléments s'étendant de manière oblique pour se déployer vers l'extérieur à l'écart de l'axe longitudinal dans la seconde configuration lorsqu'ils sont exposés de manière distale relativement à l'extrémité distale de la gaine externe.

7. Appareil selon l'une quelconque revendication précédente, le premier ensemble circuit flexible comprenant en outre :
(i) un premier sous-ensemble circuit flexible (240), le premier sous-ensemble circuit flexible comportant :
(A) un premier substrat flexible, et
(B) au moins certaines électrodes de la pluralité d'électrodes positionnées le long du premier substrat flexible, et
(ii) un deuxième sous-ensemble circuit flexible (260), le deuxième sous-ensemble circuit flexible comportant :
(A) un deuxième substrat flexible, le deuxième substrat flexible étant fixé en apposition avec le premier substrat flexible pour former un stratifié multicouche, et
(B) une ou plusieurs caractéristiques électriques configurées pour générer des signaux, la ou les caractéristiques électriques étant positionnées le long du deuxième substrat flexible.

8. Appareil selon la revendication 7, le premier ensemble circuit flexible comprenant en outre un troisième sous-ensemble circuit flexible, le troisième sous-ensemble circuit flexible (290) comportant :
(A) un troisième substrat flexible, le troisième substrat flexible étant fixé en apposition avec le deuxième substrat flexible pour former un stratifié multicouche, avec le deuxième substrat souple étant interposé entre le premier substrat flexible et le troisième substrat flexible, et
(B) au moins certaines électrodes supplémentaires de la pluralité d'électrodes, les au moins certaines électrodes supplémentaires étant positionnées le long du troisième substrat flexible.

9. Appareil selon l'une quelconque revendication précédente, l'effecteur terminal comprenant en outre un deuxième ensemble circuit flexible (450), le deuxième ensemble circuit flexible comprenant :
(i) une pluralité d'éléments s'étendant de manière oblique (458), les éléments s'étendant de manière oblique du deuxième ensemble circuit flexible étant configurés pour effectuer une transition entre une première configuration et une seconde configuration, les éléments s'étendant de manière oblique du deuxième ensemble circuit flexible étant configurés pour s'ajuster au sein d'une gaine externe dans la première configuration, les éléments s'étendant de manière oblique du deuxième ensemble circuit flexible étant configurés pour se déployer vers l'extérieur à l'écart de l'axe longitudinal dans la seconde configuration lorsqu'elles sont exposées de manière distale relativement à l'extrémité distale de la gaine externe, et
(ii) une pluralité d'électrodes (462) positionnées sur au moins certains des éléments s'étendant de manière oblique du deuxième ensemble circuit flexible, les électrodes du deuxième ensemble circuit flexible étant positionnées pour entrer en contact avec l'un ou les deux parmi les tissus ou le sang.

10. Appareil selon l'une quelconque revendication précédente, l'élément de base et la pluralité d'éléments s'étendant de manière oblique étant configurés pour s'étendre le long d'un plan plat lorsqu'ils sont exposés de manière distale relativement à l'extrémité distale de la gaine externe.

11. Appareil selon la revendication 1, dans lequel le premier élément de base et la première pluralité d'éléments s'étendant de manière oblique sont configurés pour s'étendre le long d'un premier plan plat lorsqu'ils sont exposés de manière distale relativement à l'extrémité distale de la gaine externe, dans lequel l'appareil comprend en outre :
(i) un deuxième ensemble circuit flexible (450), le deuxième ensemble circuit flexible comprenant :
(A) un second élément de base (454) s'étendant de manière distale à partir de l'extrémité distale de l'ensemble tige de cathéter,
(B) une seconde pluralité d'éléments s'étendant de manière oblique (458) s'étendant de manière oblique à partir de l'élément de base, la seconde pluralité d'éléments s'étendant de manière oblique étant configurés pour se déployer vers l'extérieur à l'écart de l'axe longitudinal dans la seconde configuration lorsqu'ils sont exposés de manière distale relativement à l'extrémité distale de la gaine externe, le second élément de base et la seconde pluralité d'éléments s'étendant de manière oblique étant configurés pour s'étendre le long d'un second plan plat lorsqu'ils sont exposés de manière distale relativement à l'extrémité distale de la gaine externe, et
(C) une seconde pluralité d'électrodes (462) positionnées sur la seconde pluralité d'éléments s'étendant de manière oblique, la seconde pluralité d'électrodes étant positionnées pour entrer en contact avec l'un ou les deux parmi les tissus ou le sang.

12. Appareil selon l'une quelconque revendication précédente, le premier ensemble circuit flexible comprenant en outre une pluralité de régions de flambage prédéfinies (520, 522, 524), les régions de flambage prédéfinies étant configurées pour flamber en réponse à la rétraction de l'effecteur terminal dans la gaine externe.
